# EUROPEAN PATENT SPECIFICATION

(11) **EP 3 760 142 B1**
(45) Date of publication and mention of the grant of the patent: **11.12.2024**
(21) Application number: 20187534.1
(22) Date of filing: 12.07.2012
(51) Int. Cl.: A61B 17/17, A61F 2/46, A61B 90/11, A61B 17/56, A61B 34/10, A61F 2/30

(54) **SURGICAL FIXTURE FOR POSITIONING AN ALIGNMENT ELEMENT**
CHIRURGISCHE VORRICHTUNG ZUM POSITIONIEREN EINES AUSRICHTUNGSELEMENTS
INSTRUMENT CHIRURGICAL SERVANT À POSITIONNER UN ÉLÉMENT D'ALIGNEMENT

(30) Priority: 12.07.2011 EP 11173606; 12.07.2011 US 201113180688
(43) Date of publication of application: 06.01.2021
(62) Divisional of application: 12740926.6
(73) Proprietor: Materialise NV, 3001 Leuven (BE)
(72) Inventor: HANANOUCHI, Takehito, Amagasaki, Hyogo 661-0967 (JP); VANGENEUGDEN, Dieter, 3900 Overpelt (BE)
(74) Representative: EIP

(56) References cited:
- WO-A1-2010/124164
- WO-A1-2011/029911
- WO-A1-2011/060536
- US-A- 5 976 149
- US-A1- 2006 058 809
- US-A1- 2006 161 167
- US-A1- 2010 016 984
- US-A1- 2010 082 035
- US-A1- 2010 087 829

## Description

### FIELD OF THE INVENTION

The present invention relates to surgical instruments for positioning an alignment element based on pre-operational planning, as well as to methods for the manufacture thereof.

### BACKGROUND

In most joint arthroplasty, replacement and/or reconstruction surgery procedures, and in particular in hip and shoulder joint surgery, a joint is replaced by a prosthetic implant. The main goal of such interventions is to relieve (arthritic) pain and/or to restore severe physical joint damage. When prosthesis fails, a revision surgery is carried out. However, this procedure is technically more difficult and time-consuming than the primary intervention and the outcome is often less satisfactory, both because there is less bone stock to work with and because the removal of adherent cement or prosthetic components may result in fracture or perforation of the bone. Furthermore, with each successive joint revision, the risk of infection and symptomatic loosening of the prosthesis may increase substantially. Accordingly, one of the most important aspects of joint surgery procedures is the correct, accurate and stable placement of the primary implant.

Correct implant placement is important in shoulder surgery, and particularly important in hip surgery. The majority of acetabular implants used in hip surgery are currently placed using the press-fit technique. In this technique, the patient's acetabulum is first reamed with a sequence of hemispherical reamers with increasing diameters, such that a hemispherical cavity is created at the location where the implant should be placed. The final (largest) reamer typically has a diameter smaller than that of the implant. In a further step, the implant is attached to an impactor and placed upon the pelvis of the patient, such that the implant supports on the rim of the reamed cavity and the orientation of the implant is anatomically suitable. Finally, the impactor is hit with a hammer until the implant sits inside the reamed cavity. Thereafter, the implant is released from the impactor.

The general consensus in the field is that the orientation of the implant determines the success of the surgery and the lifespan of the implant (Hayakawa et al., Archives of orthopedic and trauma surgery Vol. 129 (2009):1151-1156). However, the current procedure shows several shortcomings for obtaining a good orientation. Indeed, the only anatomical visual reference during final placement is the orientation of the transverse ligament (Pearse et al., Hip international Vol. 18 (2008):7-10), to which the top plane of the implant should be oriented in parallel. Accordingly, rotation around the axis of the transverse ligament remains a variable parameter. In addition, the transverse ligament is generally obscured from the surgeon's view, further hampering the orientation process. Furthermore, the impactor and hammer are both rather bulky, making it difficult to keep the impactor in a stable orientation.

Few practical solutions have been proposed for these problems. US patent application 2009/0163922 (Meridew, Metzger) describes a patient-specific guide to be positioned and optionally attached to the acetabular rim, designed to interface with the impactor so as to enforce the correct orientation. However, it is very doubtful whether such a device could be able to withstand the momentum applied to it during impaction.

US patent application 2010/0082035 and International patent application WO 2011/060536 disclose patient-specific surgical instruments for facilitating implantation of an acetabular cup prosthesis in a bone of a patient. The guides may be used for positioning a guide pin bone guide pin to the patient's acetabulum. However, such guides only provide a limited accuracy. US2006/0058809 relates to preparing a glenoid surface.

Accordingly, there is a need for alternative and improved surgical devices, and in particular surgical guiding instruments, which provide the ability to correctly and accurately insert, place and orient an implant into a patient's joint.

### SUMMARY OF THE INVENTION

The present invention is directed to a patient-specific surgical fixture for positioning an alignment element, such as a pin, a wire, a screw or a drill, on or near a glenoid socket of a shoulder joint of a patient, as defined in claim 1. The present invention is also directed to a method of manufacturing such a patient-specific surgical fixture as defined in claim 14. Embodiments of the invention are recited in the dependent claims. The present disclosure relates to surgical instruments for use in arthroplasty. The instruments are intended for facilitating surgery on ball-and-socket joints in the human or animal body. In surgery on the human body, the instruments are therefore useful for hip and shoulder joint surgery, particularly for the positioning of an acetabular cup implant or glenoid implant. The surgical instruments allow for positioning of an alignment element, where the desired position and orientation of the alignment element is typically based on pre-operational planning. The alignment element can be an indicator pin, wire, screw or drill, which acts as a navigator for the surgeon to address an optimal pre-operationally planned implant alignment direction, in the reaming and/or the impacting phase of the surgical procedure.

The surgical instruments according to the present disclosure are surgical fixtures. In a first aspect, the present invention provides surgical fixtures for positioning an alignment element. The fixtures according to the present disclosure comprise one or more patient-specific contact elements which together fit onto areas on a socket of a ball-and-socket joint, onto areas around said socket and/or onto the rim of said socket in at least three contact points. Where the socket is a glenoid cavity, the areas around the socket may include the glenoidal rim and the periglenoidal region (e.g. infraglenoidal tuberculum, supraglenoidal tuberculum and collum scapulae, etc.) and might include the acromion and processus coracoideus (coracoid process). Where the socket is an acetabulum, the areas around the socket may include the periacetabular region (e.g. the limbus acetabuli, sulcus supra-acetabularis, superior ramus, etc.).

In particular embodiments, the contact points have an arrangement wherein the angle between the line connecting one contact point and the center of the circle or ellipse best fitting the socket rim and the line connecting the adjacent contact point and said center is never greater than 180°. The surgical fixtures further comprise a positioning element which is rigidly attached to the fixture. This positioning element is provided with one or more holes which allow the insertion of the alignment element. Additionally, in particular embodiments, the positioning element is detachable from the rest of the fixture.

In particular embodiments, one (of the) contact element(s) is positioned on the fixture such that, when positioned on the bone, it interacts with an anatomical feature present on the rim of the socket or on the bone in or around the socket. In further embodiments, this anatomical feature is the posterior notch of the transverse ligament, or the coracoid process.

In particular embodiments, the disclosure provides patient-specific surgical fixtures for positioning an alignment element on the acetabular socket of a pelvic bone, comprising a one-piece structure with one or more patient-specific contact elements which fit onto one or more discrete areas on the socket. More particularly these one or more contact elements fit around and/or onto the acetabular rim, whereby at least one of said one or more contact elements interact with the posterior notch of the transverse ligament of the rim of said acetabulum so as to ensure a tight fit of said fixture on the acetabular rim. The fixture further comprises a positioning element which can be or is rigidly attached to said one-piece structure comprising contact elements, which positioning element is provided with one or more holes which allows the insertion of said alignment element.

In certain embodiments of the fixtures described herein, the surgical fixtures comprise at least two contact elements, or at least three contact elements. Together, the contact elements fit onto areas on the socket, around the socket and/or on the socket rim in at least three contact points, whereby the contact points have an arrangement wherein the angle between a line drawn between one contact point and the center of the circle or ellipse best fitting the socket rim and a line drawn between the adjacent contact point and said center is never greater than 180°. In further embodiments, the surgical fixture comprises at least two contact elements, wherein the positioning element corresponds to one of the contact elements.

In certain embodiments of the fixtures described herein, the one or more holes in the positioning element allow the insertion of said alignment element outside of said socket.

In certain embodiments of the fixtures described herein, the positioning element is detachable from the rest of said fixture.

In certain embodiments of the fixtures described herein, the one or more contact elements which fit onto areas on the rim of said socket in at least three contact points are irreversibly fixed to each other.

As indicated hereabove, the positioning element may be detachable from the rest of the fixture. In particular embodiments, the connection between the positioning element and the rest of the fixture is adapted or weakened, such that the positioning element can be detached from the rest of the fixture by breaking said connection with surgical cutting elements. In other embodiments, the connection between the positioning element and the rest of the fixture is ensured by an element selected from a dovetail coupling, interlocking features, a pinned system and a snap-fit mechanism.

In particular embodiments, the surgical fixtures according to the present disclosure further comprise a connecting structure, wherein the positioning element and/or one or more of the one or more contact elements extend from the connecting structure.

As indicated hereabove, the positioning element comprises one or more holes. In certain embodiments, the position and/or direction of at least one hole is in accordance with pre-operational planning. In certain embodiments, at least one hole is part of a drill guide. In certain embodiments, the positioning element comprises a first and a second hole with a different diameter, wherein the first hole allows the insertion of the alignment element and wherein the second hole allows the insertion of a fixation element. In further embodiments, the positioning element comprises two or more holes which allow the insertion of a fixation element. In particular embodiments, the alignment element is selected from the group comprising a pin, a wire, a screw and a drill.

In particular embodiments, the surgical fixtures according to the present disclosure are manufactured via additive manufacturing.

In a further aspect, the present invention provides methods for the manufacture of the patient-specific surgical fixtures according to the present disclosure. The methods comprise the steps of:
i. obtaining volume information of the socket of a ball-and-socket joint from a patient;
ii. obtaining the installation direction of a socket implant for the patient;
iii. identifying and selecting parts of the bone surrounding the implant zone which are suitable for inserting an alignment element;
iv. identifying and selecting parts of the bone in or surrounding the implant zone which are suitable for use as a base for the contact surface or surfaces of the surgical fixture;
v. designing and producing a surgical fixture based on the information obtained in steps i, ii, iii, iv and v.

In a further aspect, the present invention provides methods for guiding a socket implant in a socket of a ball-and-socket joint, comprising the steps of:
1) positioning a surgical fixture according to the present invention onto the socket;
2) using one of the holes provided by the fixture to insert a wire, pin, drill or screw into the bone surrounding the socket;
3) removing the surgical fixture from the socket;
4) using the wire, pin, drill or screw to guide the implant in the correct direction onto the socket according to the pre-operational planning.

In particular embodiments, step 1) involves a rotational movement of the fixture to obtain the desired orientation onto the socket and step 3) comprises detaching the positioning element from the rest of the fixture, and optionally from the bone, so as to allow thereafter, a reversal of the rotational movement of step 1) to remove the rest of the fixture from the bone.

The patient may be an animal or human patient. Therefore the socket may be any socket of a ball-and-socket joint in an animal or human body. In human patients, the socket of a ball-and-socket joint may be an acetabulum or a glenoid cavity. In particular embodiments, the socket is an acetabulum. In other embodiments, the socket is a glenoid cavity.

The surgical fixtures according to the present invention allow for a fast and accurate positioning of an indicator pin, wire, screw or drill, and allow for an efficient removal of the fixture from the anatomy after use.

### BRIEF DESCRIPTION OF THE DRAWINGS

The following figures of specific embodiments of the disclosure are merely exemplary in nature and are not intended to limit the present teachings, their application or uses. Throughout the drawings, corresponding reference numerals indicate like or corresponding parts and features.

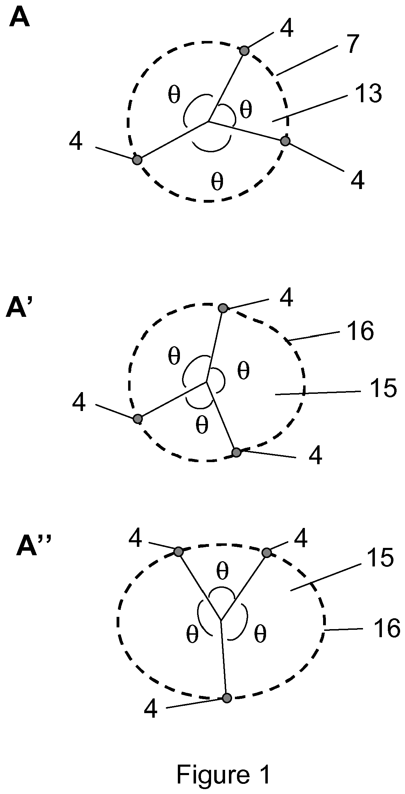

Figure 1 A, A', A": Schematic representation of the relative position of patient-specific contact points (4) and their orientation relative to the acetabular rim (7) or glenoid cavity rim (16) and the acetabulum (13) or glenoid cavity (15) according to a particular embodiment of the present invention. B, B', B", C, C', C": Schematic representation of the arrangement and shape of patient-specific contact surfaces (14) and their orientation relative to the acetabular rim (7) or glenoid cavity rim (16) and the acetabulum (13) or glenoid cavity (15) according to a particular embodiment of the present invention.

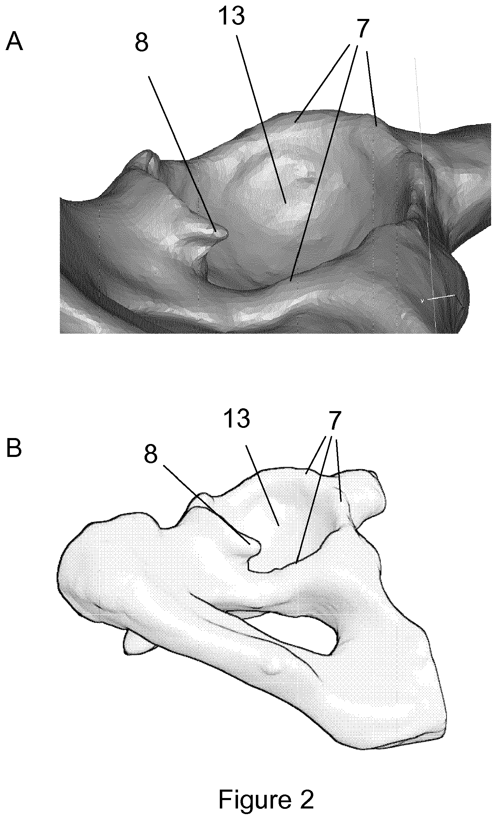

Figure 2 A, B: Drawing of an acetabulum (13) surrounded by the acetabular rim (7) and the posterior notch of the transverse ligament (8).

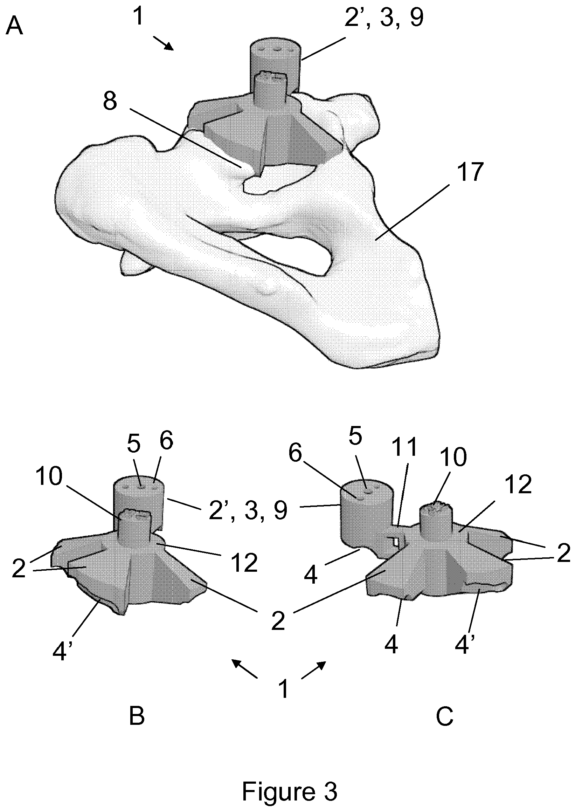

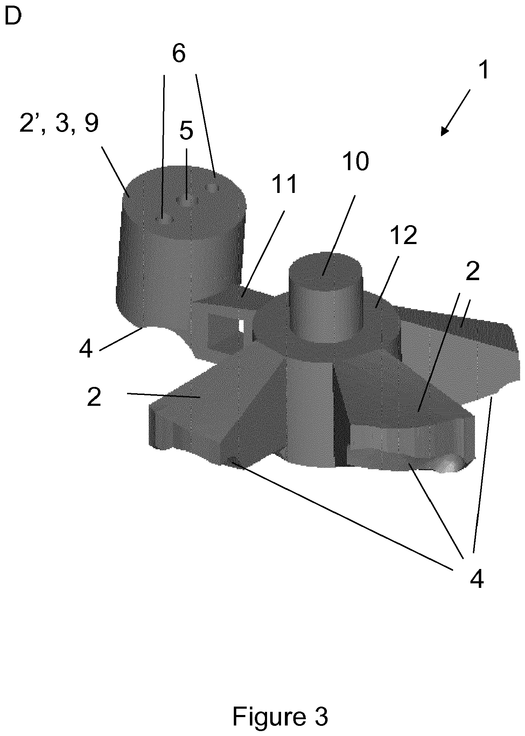

**Figure 3** Surgical fixture (1) according to a particular embodiment of the present disclosure, positioned onto an acetabulum (A), and not positioned on a bone (B, C). The fixture comprises a central connecting structure (12), a positioning element (3) and four contact elements (2, 2'), each comprising a contact surface (4). The fixture also comprises an extension (10) for manipulation of the fixture. The positioning element (3) also functions as a drill guide (9), comprises holes (5, 6) and is connected to the rest of the fixture via a weakened connection (11).

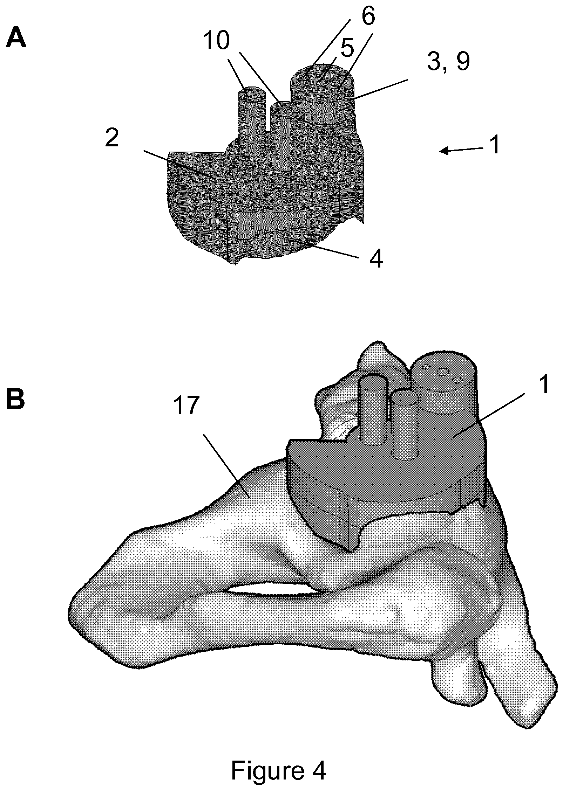

**Figure 4** A: Surgical fixture (1) according to a particular embodiment of the present disclosure, comprising a positioning element (3) and a contact element (2) comprising a contact surface (4). The fixture further comprises two extensions (10). The positioning element (3) is also a drill guide (9), comprises holes (5, 6). B: the same surgical fixture (1) positioned on an acetabulum of a pelvic bone (17).

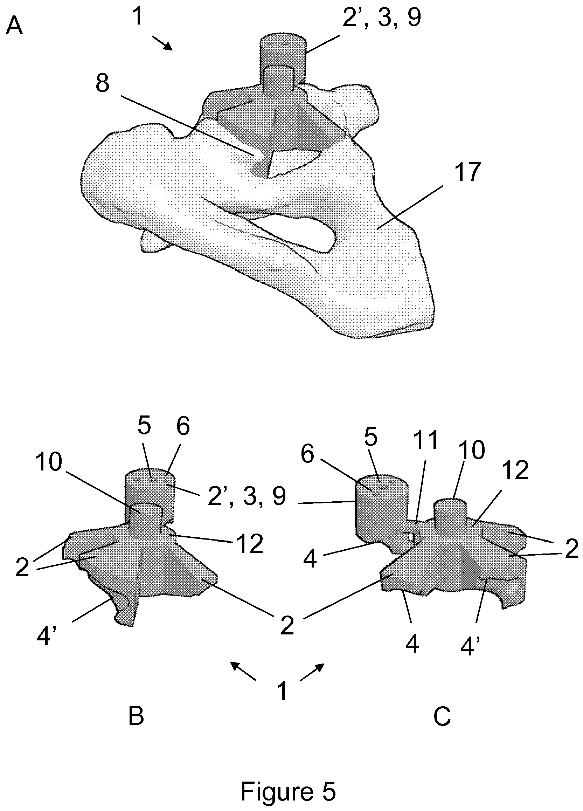

**Figure 5** A: surgical fixture (1) according to a particular embodiment of the present invention, positioned on an acetabulum (7) of a pelvic bone. B, C: surgical fixture (1) according to a particular embodiment of the present disclosure, not positioned on a bone

In the figures, the following numbering is used:
1 - surgical fixture; 2, 2' - contact element; 3 - positioning element; 4, 4' - contact point; 5 - hole for insertion of alignment element; 6 - hole for fixation element; 7 - acetabular rim; 8 - posterior notch of the transverse ligament; 9 - drill guide; 10 - extension; 11 - adapted connection; 12 - connecting structure; 13 - acetabulum; 14 - contact surface; 15 - glenoid cavity; 16 - glenoid cavity rim; 17 - pelvic bone

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of also include the term "consisting of.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1 % or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The present disclosure provides in a patient-specific surgical fixture for positioning an alignment element. The alignment element may be used for indicating a (pre-operationally planned) direction and/or position for an implant. The fixture comprises one or more patient specific contact elements, which fit onto areas on or around a socket, and/or on the socket rim; the fixture further comprises a positioning element for positioning the alignment element.

The present disclosure relates to the field of implant surgery, more particularly implants which are placed into a socket of a ball-and-socket joint. For human patients, this is an acetabular cup implant and/or a glenoid implant. The term "acetabular cup implant" as used herein refers to the component of a prosthetic hip implant which is placed into the acetabulum of a patient. The acetabulum is a concave surface of the pelvis, where the head of the femur meets with the pelvis, thus forming the hip joint. The term "glenoid implant" as used herein refers to a component of a prosthetic shoulder implant which is placed into or onto the glenoid cavity of a patient. Such implants may be used in a (total) shoulder arthroplasty or reverse (total) shoulder arthroplasty. The glenoid cavity, also known as glenoid fossa (of the scapula), is a shallow surface, which is located on the lateral angle of the scapula. This cavity forms the glenohumeral joint along with the humerus.

The terms "rim" and "socket rim" as used herein refer to the edge of a socket. Usually, this is a substantially convex edge of the concave bone surface which forms the socket. For human patients, particular examples are the acetabular rim and/or the glenoid rim. The term "acetabular rim" as used herein refers to the edge of the acetabulum, more particularly the substantially convex edge of the concave surface of the pelvis which forms the acetabulum. The term "glenoid rim" as used herein refers to the edge of the glenoid cavity, more particularly the substantially convex edge of the concave surface of the scapula which forms the glenoid cavity.

The term "socket" as used herein in the context of a ball joint, refers to a socket of a ball-and-socket joint of the human or animal body. For human patients, typical examples include the acetabulum and/or the glenoid cavity.

The term "alignment element" as used herein refers to an element which facilitates the correct positioning of an implant into or onto an anatomical socket, for example by indicating a certain location and/or direction for positioning and/or by physically guiding the implant or an implant guide to a certain location. Without such element, the implant may be positioned incorrectly, leading to suboptimal functioning of the prosthesis and discomfort to the patient.

The terms "surgical fixtures" and "fixture" as used herein refer to (patient-specific) surgical tools that can be positioned onto an anatomical part of a patient and that help a surgeon in the positioning of an alignment element.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the present disclosure provides surgical instruments for facilitating the positioning of an implant into or onto a socket of a ball-and-socket joint in the body of an animal or human patient. More particularly, in the context of humans, the present invention provides surgical fixtures for positioning an alignment element, which can be used for positioning an acetabular cup implant or a glenoid implant. The present invention is however equally useful for use in animals.

The surgical fixtures according to the present disclosure comprise at least one patient-specific contact element, i.e. a part of the surgical fixture which is used to ensure the correct positioning of the surgical fixture by contacting specific locations on the patient's anatomy.

The (one or more) patient-specific contact elements allow the surgeon to obtain the correct position of the surgical fixture onto the socket and/or socket rim, according to pre-operational planning. Indeed, the one or more contact element(s) (together) fit onto specific areas on or around the socket and/or socket rim (with or without cartilage or other soft tissue). In particular embodiments, the one or more contact elements fit onto the rim of the socket in at least three contact points. More particularly, if the fixture comprises only one contact element, that contact element fits onto specific areas on or around the socket and/or socket rim in at least three contact points. Alternatively, if the fixture comprises two or more contact elements, the contact elements are arranged such that together, they fit onto specific areas on or around the socket and/or socket rim in at least three contact points. The contact points have an arrangement, preferably so as to surround the socket, whereby the angle between:
- the line connecting one contact point and the center of the circle or ellipse best fitting the rim of the socket, and
- the line connecting the adjacent contact point and the center of the circle or ellipse best fitting the rim of the socket,
is never greater than 180°.
In particular embodiments, the angle between the contact points (as determined by lines connecting each contact point with the center of the circle of ellipse) is never greater than 175, 170, 165, 160, 155, 150, 145, 140, 135, 130, 125, 120, 115, 110, 105, 100, 95 or 90°.
This will be explained in further detail herein below.

In particular embodiments, the contact elements which extend over these three contact points are irreversibly attached to each other. In particular embodiments, at the least the part of the fixture comprising these contact elements is made as a one piece structure. This implies that the contact elements are fitted onto the rim of the socket simultaneously.

The surgical fixtures according to the present disclosure further comprise a positioning element. The positioning element is a part of the fixture which is used for placing the alignment element onto or into the bone in or surrounding the socket in a pre-operationally planned position. In particular embodiments, the positioning element is placed such that it guides the alignment element in the bone surrounding the socket. The bone surrounding the socket suitable for placing the alignment element is, for example, the bone in the periacetabular region (e.g. the limbus acetabuli, sulcus supra- acetabularis, superior ramus, etc.) or periglenoidal region (e.g. infraglenoidal tuberculum, supraglenoidal tuberculum and collum scapulae, etc.). Therefore, the positioning element is provided with at least one hole or slit which either guides or allows the insertion of an alignment element. The positioning element is either integrated in or rigidly attached to the rest of the fixture comprising the contact elements. In particular embodiments, the positioning element is a structure rigidly attached to the contact elements. In these embodiments, the contact element(s) and the positioning element in the surgical fixture, allow for the correct positioning of the fixture on the bone and thus the correct positioning of an alignment element. Indeed, the fixed position of the positioning element relative to the contact element(s) and the rigidity of the fixture ensure that once secured in the right position on the bone via the contact element(s), the positioning element allows the accurate positioning of an alignment element, according to pre-operational planning.

The positioning element is attached rigidly to the fixture. Thus the connection between the positioning element and the rest of the fixture is not flexible or resilient. This means that the relative position of the positioning element and the contact element(s) of the fixture is fixed, as long as the positioning element is attached to the fixture. Accordingly, the position and orientation of the positioning element on the socket is determined by the position of the contact elements on the bone. Moreover, this ensures that the relative position of the positioning element and the contact element(s) remains fixed during positioning the fixture on the socket joint, and therefore ensures an accurate positioning of the alignment element via the positioning element, according to pre- operational planning.

In particular embodiments, the fixtures of the present disclosure are characterized by the fact that the positioning element of the fixture is detachable, i.e. detachable from the rest of the surgical fixture. This can be ensured by the fact that the positioning element is attached to the surgical fixture by a rigid connection which is weakened and/or adapted such that it can be broken when applying force, or cut or removed using surgical instruments. Additionally or alternatively, the positioning element may be removably attached to the rest of the surgical fixture via features which allow a rigid, reversible connection of the positioning element, e.g. interlocking features, a dovetail coupling, a pinned system, a snap-fit system and the like. The fact that the positioning element is detachable facilitates the removal of the surgical fixture from the bone after the alignment element is introduced into the bone through the positioning element.

The desired position of the alignment element for guiding the placement of an implant is determined by pre-operative planning. Moreover, using preoperative planning optimal contact points for the contact element(s) of the surgical fixture according to the present invention can be determined. Indeed, while the number and shape of the contact elements may vary, the contact points comprised therein determine the stability of the fixture. The optimal position of the contact points ensures positional stability of the surgical fixture onto the socket. Optimally, to ensure positional stability of the fixture onto an acetabulum, the contact points have a circular or substantially circular arrangement, preferably so as to surround the acetabulum (13), wherein the angle e defined by two adjacent contact points is never greater than 180°. In other words, the angle between the line connecting one contact point (4) and the center of the circle best fitting the acetabular rim (7) and the line connecting the adjacent contact point (4) and said center is never greater than 180°. This is shown in Figure 1 A. Hereby the acetabulum is considered to have a substantially circular shape.

The glenoid cavity on the other hand, can be considered piriform. Thus, the glenoid cavity comprises a substantially circular shape on one side, but tapering towards the other side. Therefore, to ensure positional stability of the fixture onto a glenoid cavity (15), the contact points have a circular or substantially circular arrangement around the circumference of the rim of the glenoid cavity. More particularly, the angle θ formed by a first line drawn between one contact point (4) and the center of the circle best fitting the (substantially circular part of) the glenoid cavity rim (16) and a second line drawn between the adjacent contact point (4) and said center is never greater than 180°. This can also be expressed in terms of the sector angle defined by two adjacent contact points (4) which is never greater than 180°. This is shown in Figure 1 A'.

Alternatively, the glenoid cavity may be considered as having a roughly elliptical shape. In that case, to ensure positional stability of the fixture onto a glenoid cavity (15), the contact points have an arrangement, wherein the angle e between the (straight) line connecting one contact point (4) and the center of the ellipse best fitting the glenoid cavity rim (16) and the (straight) line connecting an adjacent contact point (4) and said center is never greater than 180°. Again this can be expressed by the fact that the sector angle defined by two adjacent contact points is never greater than 180°. This is shown in Figure 1 A".

Thus, more generally for an undefined socket, in particular embodiments of the fixtures of the present disclosure, the contact elements fit onto the socket and/or socket rim by contacting said socket and/or rim at different contact points. Together, the contact elements fit onto areas of (or around) a socket and/or a socket rim in at least three contact points, wherein the contact points have an arrangement, preferably so as to surround the socket, wherein the angle between a line drawn between one contact point and the center of the circle or ellipse best fitting the socket rim and a second line drawn between an adjacent contact point and said center is never greater than 180° or similarly the sector angle defined by two adjacent contact points is never greater than 180°. These contact points may all be located on the same contact element (i.e. one contact element comprises these three contact points), or distributed over two or more contact elements.

In certain embodiments, the one or more contact elements of the surgical fixtures according to the disclosure contain, on the surface which is intended for placement on the bone (with or without cartilage or other soft tissue), patient-specific surfaces, i.e. anatomy engagement surfaces which at least partially match the surface of (or around) the socket and/or the socket rim. This implies that the contact element comprises of a plurality of contact points (corresponding to a contact surface). The positional stability of the surgical fixture onto the acetabulum is then at least in part determined by the size and position of the contact surfaces on the contact element(s). The patient specific surface of a contact element typically spans an area which varies between one square micrometer (µm²) and fifty square centimeters (cm²).

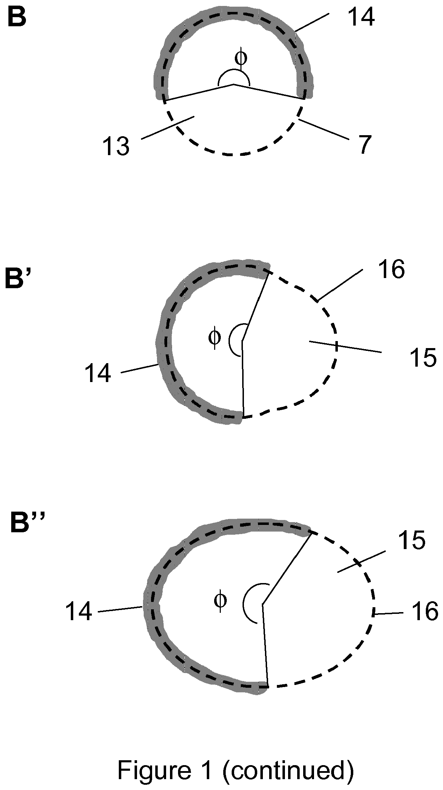

In particular embodiments, the surgical fixtures according to the disclosure comprise only one contact element, which has a single contact surface. In particular embodiments, this contact surface (14) spans the surface of the socket (13) and/or socket rim (7) over an angle q, of at least 180°, as schematically drawn for a circular socket (acetabulum) in Figure 1 B. A piriform or elliptical socket (glenoid cavity) imposes a similar requirement (Figures 1 B' and B").

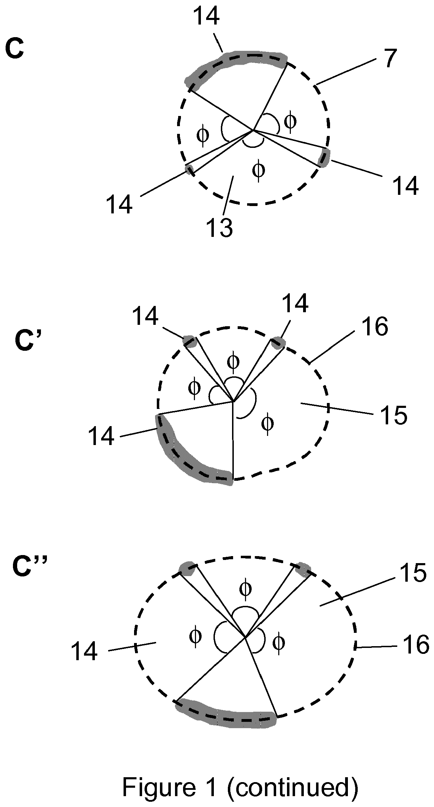

Alternatively, the surgical fixtures according to the invention may comprise more than one contact element comprising a patient-specific contact surface. A stable positioning of the surgical fixture can then optimally be obtained if the contact surfaces of the different contact elements have an arrangement, on or around the socket (13) and/or socket rim (7), wherein for every pair of adjacent contact points not belonging to the same contact surface (14), the angle q, between the line drawn between one contact point and the center of the circle best fitting the socket rim (7) and a second line drawn between the adjacent contact point and said center is never greater than 180°, as schematically drawn for a circular socket (acetabulum) in Figure 1 C. Again, a piriform or elliptical socket (glenoid cavity) imposes a similar requirement (Figure 1 C' and C"). Also fixtures comprising combinations of one or more contact points with one or more contact surfaces are envisaged.

Alternative or specific combinations of the above are also envisaged. In certain embodiments, as indicated above, the surgical fixtures according to the present disclosure comprise only one contact element, which comprises several patient-specific contact points and/or contact surfaces. In other embodiments, the surgical fixtures according to the present disclosure comprise at least two contact elements, each comprising one or more patient-specific contact points and/or surfaces as described hereabove. In certain embodiments, the surgical fixtures according to the present disclosure comprise three contact elements, each comprising one or more patient-specific contact points and/or surfaces as described hereabove. In further embodiments, the surgical fixtures according to the present disclosure comprise four contact elements, each comprising one or more patient-specific contact points and/or surfaces as described hereabove. In yet further embodiments, the surgical fixtures according to the present invention comprise (at least) five, six, seven, eight, nine, ten or more contact elements, each comprising one or more patient-specific contact points and/or surfaces as described hereabove.

It will be understood to the skilled person that, in the fixtures as described herein the different contact elements of the surgical fixtures of the present invention need not make contact with the bone (with or without cartilage or other soft tissue) in the same way and need not contact the bone over their entire surface. Thus, in particular embodiments, at least one contact element contacts, at least partially, the socket rim via its contact point(s) and/or surface(s). In further embodiments, all contact elements contact, at least partially, the socket rim via the contact point(s) and/or surface(s) comprised therein. Specifically, in particular embodiments, the position and/or orientation of at least one contact element on the surgical fixture is patient-specific.

Advantageously, at least one contact element is positioned on the fixture such that its patient-specific surface corresponds to the surface of the corresponding socket in the location of a conspicuous anatomical feature in or around the socket. For the acetabulum, this is for example the posterior notch of the transverse ligament, hereinafter also referred to as "posterior notch". The posterior notch of the transverse ligament (8) of the acetabulum (13) is drawn in Figure 2 A and B. For the glenoid cavity, this is for example the coracoid process (processus coracoideus); this is a small hook- like structure on the lateral edge of the superior anterior portion of the scapula.

The provision of a contact element which fits into or against such a feature such as the posterior notch or the coracoid process ensures a stable fixation of the surgical fixture onto the socket. For instance, in particular embodiments, the fixture of the present disclosure is provided with a contact element fitting onto and/or into the posterior notch. The device can be designed such that, in order to place the contact element within the notch, the surgical fixture according to the invention must be placed on the acetabulum in a first position whereby the contact element is placed next to the posterior notch. The desired final position of the surgical fixture onto the acetabulum is then obtained via a (slight) rotational movement of the fixture, which allows the contact element to move over and optionally into the notch until it reaches the rim of the notch. In particular embodiments, the contact between the contact element and the rim of the posterior notch of the transverse ligament ensures a rotational stop when the fixture is in the correct position. Thus, in particular embodiments, the fixtures of the invention, and more particularly a contact element thereof, may comprise a rotational undercut feature for registering on an anatomical feature around the socket, such as the posterior notch of the transverse ligament, or on top of the coracoid process.

Accordingly, in particular embodiments, the surgical fixtures of the disclosure are designed such that they comprise at least one contact element which interacts with an anatomical feature in the socket, on the socket rim or on the bone (with or without cartilage or other soft tissue) surrounding the socket. Where the socket is a glenoid cavity, the bone surrounding the socket includes the glenoidal rim and the periglenoidal region (e.g. infraglenoidal tuberculum, supraglenoidal tuberculum and collum scapulae, etc.) and might include the acromion and coracoid process. Where the socket is an acetabulum, the bone surrounding the socket includes the periacetabular region (e.g. the limbus acetabuli, sulcus supra-acetabularis, superior ramus, etc.). In particular embodiments, the contact element is to be positioned on or within the posterior notch or the coracoid process. Specifically, one contact element is provided on the surgical fixture such that, when correctly positioned on the bone (i.e. in its final position), it fits within or onto the posterior notch or to the location of the coracoid process. More particularly, it is envisaged that, such a contact element grabs or locks onto the posterior notch or the coracoid process. This ensures a strong rotational stop when the fixture is in the correct position and therefore an accurate positioning of the fixture on the socket. Thus, in particular embodiments the shape of one contact element and its position on the fixture is such that, when positioned on the bone, it grabs onto or around the anatomical features such as the posterior notch or the coracoid process. Thus in particular embodiments such a contact element may comprise an undercut or may even be hook- shaped, such that it can grab on or around the anatomical feature. Thus, in particular embodiments one contact element comprises an undercut, or comprises a hook-shaped feature. In particular embodiments this undercut specifically mates with the acetabular notch.

In particular embodiments as described above, the one or more contact elements are designed such that correct positioning can be ensured by rotation of the contact elements over the rim of the socket. Thus, after placement of the contact elements on the bone, a slight rotation of the fixture will ensure that the contact elements fit into the planned position. Through the provision of an undercut in only one direction, the fixture can be rotated into position despite the fact that the contact elements are rigidly fixed to each other.

In further particular embodiments, it is envisaged that the contact element which interacts with a specific anatomical feature on the rim of the socket or the surrounding bone may further also provide a locking feature. Indeed, the shape of this contact element may be adapted such that, when positioned on the bone, it snaps onto and thus in fact locks onto this feature.

The surgical fixtures of the present disclosure allow correct positioning of an alignment element by way of the positioning element. For this purpose, the positioning element of the fixtures of the disclosure comprises an opening or hole which allows the insertion of an alignment element which is guided by the positioning element into the bone surrounding the socket, such as the pelvic bone or scapula. The alignment element may be a wire, pin, screw or drill, particularly a metal wire, pin, screw or drill. In particular embodiments, the alignment element is a wire or a pin, particularly a Kirschner wire (K-wire) or a Hoffmann pin.

In particular embodiments, the positioning element ensures placement of the alignment feature outside the socket. Indeed, in order to allow the guiding of the positioning of an implant for a socket of a ball-joint, the alignment element is typically positioned on the bone surrounding the socket, for example in the periacetabular area of the acetabulum (e.g. the limbus acetabuli, sulcus supra-acetabularis, superior ramus, etc.) or periglenoidal area of the glenoid cavity (e.g. infraglenoidal tuberculum, supraglenoidal tuberculum and collum scapulae, etc.) of the patient, in a direction parallel to the installation direction of the implant. For instance, for the positioning of an acetabular cup implant, the alignment element is typically positioned on the pelvic bone in a direction parallel to the installation direction of the acetabular implant. However, in particular embodiments, the positioning element ensures placement of the alignment feature inside (typically centrally in) the socket.

The optimal orientation of the opening of the positioning element can be obtained by determining the orientation of the positioning element according to pre-operational planning. Thus, in preferred embodiments, the direction and/ or the position of at least one hole of the surgical fixtures of the invention which allows the insertion of an alignment element is in accordance with pre-operational planning. More particularly, this implies that the relative position and/or orientation of least one hole relative to the contact element(s) of the surgical fixtures of the invention is in accordance with pre- operational planning. This allows the use of standard alignment elements such as K- wires. The positioning and/or orientation of the hole can be obtained via a certain location and/or orientation of the positioning element relative to the rest of the surgical fixture, via a certain location and/or position of the hole in the positioning element, or via a combination of the two. Additionally or alternatively, the shape of the alignment element itself can be provided such that, when inserted into the positioning element, it ensures the correct orientation to guide the implant.

Although the correct positioning of the surgical fixtures of the disclosure on the bone allows the insertion of an alignment element into the bone as soon as the correct positioning of the surgical fixture is obtained, the insertion of the alignment elements is significantly facilitated if the surgical fixtures are (temporarily) fixed to the bone and/or locked into the correct position. Thus, in particular embodiments, the surgical fixtures may comprise fixation features such as holes, which allow for fixation of the surgical fixtures onto the bone, for example using screws, wires or pins. Thus, in particular embodiments, the surgical fixtures of the invention comprise at least one hole in addition to the hole which is meant for insertion of the alignment element. In particular embodiments, the surgical fixtures of the present invention comprise at least two holes. In certain embodiments, the hole(s) used as fixation features and the hole(s) used for insertion of the alignment element are located on the positioning element. In certain embodiments, the hole(s) used as fixation features and the hole(s) used for insertion of the alignment element are cylindrical. As both types of (cylindrical) holes have a different function, they may have a different diameter. The different diameter of the holes also avoids the surgeon from inserting the alignment element into a fixation feature, especially when the fixation features have a smaller diameter than the hole(s) for inserting an alignment element.

In particular embodiments of the fixtures according to the disclosure, the positioning element and the one or more contact element(s) for positioning on the rim of the socket may be separate units which are detachable. However, when attached, the relative position between the contact element(s), the positioning element and the rest of the fixture is fixed.

In particular embodiments, the positioning element is located onto or integrated in a contact element, i.e. the structure comprising the positioning element has a surface which contacts with the bone. In particular embodiments however, the contact element is different from the one or more contact elements for placement on the rim of the socket. More particularly, it does not fit onto the rim of the socket but contacts a region outside the socket. A positioning element and such a contact element may form a single unit, thus the positioning element may also function as a contact element in that it helps to ensure accurate positioning on the bone.

In particular embodiments, the positioning element is integrated into or extends from a contact element on the rim of the socket. Where a positioning element is located onto a contact element on the rim of the socket, the hole(s) in the positioning element continue(s) through the contact element. In this way, the contact element does not block the insertion of fixation elements and/or alignment elements into the bone. Thus, in further embodiments, also the contact element(s) onto which a positioning element is located, contain at least one hole.

In some cases, the positioning of a second, or even a third alignment element allows an even more precise positioning of a socket implant. Thus, in particular embodiments, the surgical fixtures according to the present invention comprise more than one positioning element, of which at least one is detachable. In further embodiments, all positioning elements are detachable.

Insertion of the alignment element and/or fixation elements into the bone may be facilitated by first drilling a hole in the bone. The alignment element and/or fixation element(s) are then inserted into the hole drilled in the bone. Thus, in particular embodiments, at least one of the openings or holes on the positioning element (and the contact element(s) is part of a drill guide, which is located on the positioning element(s) or the contact element(s). In particular embodiments, the surgical fixture of the invention may be provided with a drill guide which can be positioned onto the hole.

In a particular embodiment, the surgical fixture according to the present disclosure is a disk (one contact element) comprising, on the side and/or edges for placement on the bone (with or without cartilage or other soft tissue), one or more contact points and/or contact surface(s) which match (i.e. specifically mate with) areas of (or around) the socket and/or the socket rim as described hereabove; and, on the opposing side of the contact surfaces, a positioning element. Advantageously, a less bulky fixture is obtained by omitting certain sectors from the disk, thus creating a surgical fixture containing two or more contact elements with one or more contact points and/or contact surfaces as described above. In particular embodiments, the contact elements may be interconnected by longitudinal structures from a central structure.

Thus, in certain embodiments, the surgical fixtures according to the present disclosure comprise a connecting structure, wherein one or more contact elements extend from the connecting structure. In further embodiments, two or more contact elements extend from the connecting structure. Also the positioning element(s) may extend from the connecting structure. In certain embodiments, the connecting structure is a central element, particularly a central axis. In particular embodiments, the contact element(s) and/or the positioning element(s) are separate units which are connectable to the connecting structure, central element or axis. In alternative embodiments, the surgical fixtures of the present disclosure are manufactured as a single piece.

In particular embodiments, the fixture may contain extensions, positioned either on the contact elements and/or a central structure connecting the contact elements. The extensions increase grip on the fixture and visibility during the positioning of the fixture on the bone. Such extensions can vary in structure such as, but not limited to rod-like structures, hooks, etc. Typically, the extensions will extend from the fixture structure in a direction which is transversal to the contact surface of the contact elements.

In particular embodiments, the fixtures of the invention contain guiding features for guiding surgical instruments. As indicated above, such guiding features may include a drill bore for guiding a drill to make a hole in the bone for the alignment element. However, additional or alternative guiding elements may also be envisaged, including on one or more of the contact elements and/or a connecting structure (where applicable). Such guiding features include holes, slits grooves etc. It can further be envisaged that attachment features are provided on one or more parts of the fixture, for fixing additional elements, such as guiding features in a reversible way.

Once the alignment element is inserted into the bone via the positioning element(s), the surgical fixture must be removed from the socket in order to proceed with the surgery, e.g. to insert the socket implant. However, the inserted alignment element (and/or fixation elements) may limit the mobility of the surgical fixture, particularly when in the final position of the fixture one of the contact elements contacts or grabs an anatomical feature such as the posterior notch or the coracoid process. To overcome this problem the present invention provides that in particular embodiments of the surgical fixtures of the invention at least one positioning element on the surgical fixture is detachable. Additionally, it is further envisaged in particular embodiments that one or more contact elements may be detachable.

In order to ensure a positioning element which is detachable from the contact elements or detachable contact elements, the present invention envisages embodiments wherein the connection between the positioning or contact element and the rest of the fixture is adapted or weakened to make the positioning or contact element detachable, while still maintaining the required rigidity to assure correct positioning. Thus, in particular embodiments, the connection between at least one positioning element or contact element and the rest of the fixture is adapted and/or weakened such that it can be detached from the guide with surgical cutting instruments. In certain embodiments, this connection is located on an extension of the connecting structure as described hereinabove.

Alternatively, the one or more positioning elements and/or contact elements may be removably connected to the rest of the fixture via a specific mechanism such as a dovetail coupling, interlocking features, a pinned system, a snap-fit system, or a combination thereof. This avoids the use of surgical cutting instruments. Moreover, if the connection between the positioning element(s) and/or the contact element(s) and the rest of the fixture is a standard connecting feature, the positioning element(s) can be reused on another surgical fixture. In certain embodiments, this connection is located on an extension of the connecting structure as described hereabove. In more particular embodiments, the positioning feature (which may itself comprise a contact surface so as to form a contact element) is connected to the remainder of the fixture comprising the one or more contact elements fitting on the rim of the socket by a standard connecting feature such as a dovetail coupling.

In particular embodiments, the surgical fixtures of the present disclosure further comprise one or more extensions on the fixture which do not contain a contact element or positioning element. Such extensions as such do not contribute to the fit of the fixture on the bone but may allow an enhanced grip of the surgeon on the surgical fixtures, and may for example facilitate the rotational movement of the surgical fixture during its positioning, or facilitate the removal of the fixture after the positioning of the alignment element. For further facilitation of the rotational movement, such extensions may comprise a screw drive, i.e. a feature that allows the extension (and thus fixture) to be turned with a screw driver, hex key, or the like. Alternatively, the screw drive is not positioned on an extension, but elsewhere on the fixture.

In a further aspect, the present disclosure provides methods for the manufacture of the surgical fixtures described herein.

The surgical fixtures according to the present disclosure comprise one or more patient-specific contact points and/or surfaces. Also the relative position and/or orientation of the positioning element(s) and/or the contact elements may be patient-specific. The generation of patient-specific surgical fixtures is done based on pre-operative images of the bone area (with or without cartilage or other soft tissue) surrounding the socket of the ball joint under consideration (e.g. the pelvic bone or the scapula), and planning of the surgery. More particularly, the generation of patient- specific surgical fixtures is done based on pre-operative images of the socket (e.g. the acetabulum or the glenoid cavity), and planning of the surgery. Accordingly, methods for producing the surgical fixtures according to the present disclosure typically comprise the steps of:
i. Obtaining volume information of a socket, e.g. the acetabulum or glenoid cavity, from a patient.
ii. Obtaining the installation direction of the socket implant for the patient.
iii. Identifying and selecting parts of the bone (with or without cartilage or other soft tissue) surrounding the implant zone which are suitable for inserting an alignment element.
iv. Identifying and selecting parts of the bone in or surrounding the implant zone which are suitable for use as a base for the contact points and/or contact surface(s) of the surgical fixture.
v. Designing and producing a surgical fixture based on the information obtained in steps i, ii and iii.

The step of obtaining volume information of the socket typically comprises obtaining digital patient-specific image information which can be done by any suitable means known in the art, such as for example a computer tomography (CT) scanner, a magnetic resonance imaging (MRI) scanner, an ultrasound scanner, or a combination of Roentgenograms. A summary of medical imaging has been described in "Fundamentals of Medical imaging", by P. Suetens, Cambridge University Press, 2002.

In a particular embodiment, Additive Manufacturing (AM) techniques are used for manufacturing the surgical fixtures according to the invention, or parts thereof. AM techniques are particularly useful to manufacture patient-specific contact surfaces, or to produce the surgical fixtures in one piece. As an example, the manufacturing of medical-image-based patient-specific surgical instruments via AM is described in US Pat. No. 5.768.134 (Swaelens et al).

AM can be defined as a group of techniques used to fabricate a tangible model of an object typically using three-dimensional (3-D) computer aided design (CAD) data of the object. Currently, a multitude of Additive Manufacturing techniques is available, including stereolithography, Selective Laser Sintering, Fused Deposition Modeling, foil- based techniques, etc.

Selective laser sintering uses a high power laser or another focused heat source to sinter or weld small particles of plastic, metal, or ceramic powders into a mass representing the 3-dimensional object to be formed.

Fused deposition modeling and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.

Typically AM techniques start from a digital representation of the 3-D object to be formed. Generally, the digital representation is sliced into a series of cross-sectional layers which can be overlaid to form the object as a whole. The AM apparatus uses this data for building the object on a layer-by-layer basis. The cross-sectional data representing the layer data of the 3-D object may be generated using a computer system and computer aided design and manufacturing (CAD/CAM) software.

The surgical fixtures according to the present disclosure may be manufactured in different materials. Typically, only materials that are biocompatible (e.g. USP class VI compatible) with the animal or human body are taken into account. Preferably the surgical fixture is formed from a heat-tolerable material allowing it to tolerate high- temperature sterilization. In the case selective laser sintering is used as an AM technique, the surgical template may be fabricated from a polyamide such as PA 2200 as supplied by EOS, Munich, Germany or any other material known by those skilled in the art may also be used.

A further aspect of the present disclosure provides methods for using the surgical fixtures described herein. More particularly, the present invention provides methods for guiding an implant in a socket of a ball joint such as an acetabulum or glenoid cavity. The methods comprise the steps of:
1) Positioning a surgical fixture as described herein onto the socket, e.g. acetabulum or glenoid cavity. In certain embodiments, this step involves a (slight) rotational movement of the surgical fixture. The rotational movement facilitates obtaining the desired (pre-operationally planned) orientation of the fixture onto the socket;
2) Optionally fixing the surgical fixture onto the socket. This step is only possible if the surgical fixture comprises holes for inserting one or more fixation elements;
3) Using one of the holes provided by the positioning element of the surgical fixture to insert a pin, wire, drill or screw into the bone surrounding the socket;
4) Removing said surgical fixture from the socket, and
5) Using the pin, wire, drill or screw of step 3) to obtain the correct implant direction; this is the direction according to the pre-operational planning.

In particular embodiments, step 1 involves a rotational movement of the fixture to obtain the desired orientation onto the socket in such a way that the contact element which interacts with an anatomical feature on the rim of the socket is fitted into place. In particular embodiments, the rotational movement of the contact elements will allow the undercut or hook of the contact element to be tightly fixed onto an anatomical feature. More particularly for fixtures on the acetabulum, the placement of the fixture is envisaged to involve the fixing of the rotational undercut of a contact element of the device onto the posterior notch of the transverse ligament.

In particular embodiments, step 4 will comprise detaching said positioning element from the rest of the fixture and from the socket, so as to allow thereafter, a reversal of the rotational movement of step 1 to remove the rest of the fixture from the socket. In particular embodiments, this is ensured by releasing the coupling feature of the positioning element (e.g. dovetail). In alternative particular embodiments, this is ensured by breaking the connection between the positioning element and the rest of the fixture.

It is noted that the alignment element envisaged in the context of the present disclosure may be used as a visual alignment element or a physical alignment element. In particular embodiments, the alignment element is a pin or wire and is used as a physical alignment element to guide an implant or implant guide onto the bone in the correct position. Typically the implant or implant guide will comprise a hole or slit which is positioned such that, when the hole or slit of the implant or implant guide is mated with the alignment feature, it will guide the implant and/or implant guide directly in the desired position on the socket of the ball joint.

The disclosure will now be illustrated by the following, non-limiting illustrations of particular embodiments of the disclosure.

### EXAMPLES

### Example 1: surgical fixture with multiple contact elements.

The surgical fixtures according to the present disclosure may comprise more than one contact element. Figure 3 A shows such a surgical fixture (1) according to a particular embodiment of the present invention, positioned onto the acetabulum of a pelvic bone (17), more particularly the pelvic bone of Figure 2 B. Figures 3 B and C show two views of the same fixture, not positioned on a bone.

The fixture comprises a positioning element (3) and four contact elements (2, 2'), each comprising a patient-specific contact surface (4). One of the contact surfaces (4') is designed to register onto the posterior notch of the transverse ligament (8), thereby providing a rotational undercut and a rotational lock. This facilitates the correct positioning of the fixture onto the acetabulum.

The positioning element forms a single unit with one of the contact elements (2'), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder. Each of the contact elements (2, 2') extends from a connecting structure (12). The connection (11) between the positioning element (3) and the connecting structure (12) is adapted, such that the positioning element can be easily detached from the surgical fixture with surgical cutting instruments. The surgical fixture (1) further comprises an additional extension (10), which allows for an enhanced grip on the fixture. The extension (10) is further provided with an identification code, for example a patient identifier.

Figure 3 D shows a similar fixture as shown in Figures 3A-C, wherein the extention (10) is not provided with an identification code.

### Example 2: surgical fixture with one contact element.

The surgical fixtures according to the present disclosure may comprise only one contact element. Figure 4 A shows such a surgical fixture (1) according to a particular embodiment of the present invention. The fixture comprises a positioning element (3) and one contact element (2). The contact element comprises a patient-specific contact surface (4). The positioning element is attached to the contact element (2), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder. The connection between the positioning element (3) and the contact element (2) is adapted (not shown), such that the positioning element can be easily detached from the surgical fixture with surgical cutting instruments. The surgical fixture (1) further comprises two extensions (10), which are located on the contact element (2) and facilitate the rotational movement of the fixture during positioning. Figure 4 B shows the same fixture (1), positioned on an acetabulum of a pelvic bone (17). The contact surface on the contact element of the fixture surrounds the acetabulum over an angle of more than 180°.

### Example 3: surgical fixture providing rotational lock.

Figure 5 A shows a surgical fixture (1) according to a particular embodiment of the present disclosure, positioned onto the acetabulum of a pelvic bone (17), more particularly the pelvic bone of Figure 2 B. Figures 5 Band C show two views of the same fixture, not positioned on a bone. The fixture comprises a positioning element (3) and four contact elements (2, 2'), each comprising a patient-specific contact surface (4, 4'). Each of the contact elements (2, 2') extends from a connecting structure (12). One of the contact surfaces (4') is designed to register onto the posterior notch of the transverse ligament (8). More particularly, the contact surface (4') is designed to partially surround the posterior notch (8), thereby providing a rotational undercut and a strong rotational lock. This significantly facilitates the correct and stable positioning of the fixture onto the acetabulum. In the illustrated embodiment, the positioning element forms a single unit with one of the contact elements (2'), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder.

In this embodiment, the connection (11) between the positioning element (3) and the connecting structure (12) is adapted, such that the positioning element can be easily detached from the surgical fixture with surgical cutting instruments. The contact element (2') which forms a single unit with the positioning element (3) has a contact surface (4) which fits a part of the acetabular rim (7), but does not provide a rotational lock, as this would potentially impede the removal of the detached positioning element (3) from the acetabulum.

The surgical fixture (1) further comprises an additional extension (10), which allows for an enhanced grip on the fixture.

## Claims

1. A patient-specific surgical fixture (1) for positioning an alignment element, such as a pin, a wire, a screw or a drill, on or near a glenoid socket of a shoulder joint of a patient, said fixture comprising:
a. a one-piece structure comprising one or more patient-specific contact elements (2); and
b. a positioning element (3) rigidly attached to said one-piece structure, provided with one or more holes (5) which allow the insertion of said alignment element,
wherein the patient-specific contact elements are configured to fit onto at least one of:
one or more areas around the glenoid socket, or
a rim of the glenoid socket,
by means of anatomy engagement surfaces, each of which at least partially match a surface of bone or cartilage of said at least one of: said one or more areas around the glenoid socket, or said rim of the glenoid socket.

2. The patient-specific fixture according to claim 1 , wherein at least one of said one or more holes is part of a drill guide (9).

3. The patient-specific fixture according to any one of claims 1 to 2, wherein the one or more patient-specific contact elements are configured to fit onto the at least one of: said one or more areas around said glenoid socket, or onto the rim of said glenoid socket in at least three contact points, wherein an angle between a line drawn between one contact point and a center of a circle or ellipse best fitting the rim or a substantially circular section of the rim of said glenoid socket and a line drawn between an adjacent contact point and said center is never greater than 180°.

4. The patient-specific fixture according to any one of claims 1 to 3, wherein a surface of at least one of said one or more patient-specific contact elements is configured to correspond to a surface of a coracoid process of the shoulder joint such that, with the anatomy engagement surface of the at least one of said one or more patient-specific contact elements being fitted onto the coracoid process so as to engage with the surface of said coracoid process, the surface of the coracoid process stops a rotation of the patient-specific surgical fixture.

5. The patient-specific fixture according to any one of claims 1 to 4, wherein the one or more holes are configured to allow the insertion of said alignment element into said glenoid socket.

6. The patient-specific fixture according to claim 5, wherein the one or more holes are configured to allow the insertion of said alignment element centrally into said glenoid socket.

7. The patient-specific fixture according to any one of claims 1 to 4, wherein the one or more holes are configured to allow the insertion of said alignment element outside of said glenoid socket.

8. The patient-specific fixture according to any one of claims 1 to 7, wherein said positioning element is detachable from the rest of said patient-specific surgical fixture,
wherein optionally a connection between said positioning element and the rest of said patient-specific surgical fixture comprises an element selected from a dovetail coupling, interlocking features, a pinned system and a snap-fit mechanism or wherein said connection is adapted or weakened, such that the positioning element can be detached from the rest of said patient-specific surgical fixture by breaking said connection with surgical cutting instruments.

9. The patient-specific fixture according to any one of claims 1 to 8, wherein the one or more patient-specific contact elements are configured to fit onto areas on the rim of said glenoid socket in at least three contact points and are irreversibly fixed to each other.

10. The patient-specific fixture according to any one of claims 1 to 9, wherein the one or more patient-specific contact elements comprises at least two patient-specific contact elements and wherein said positioning element corresponds to one of said at least two patient-specific contact elements.

11. The patient-specific fixture according to any one of claims 1 to 10, further comprising a connecting structure, wherein at least one of said positioning element or one or more of said one or more patient-specific contact elements extend from said connecting structure.

12. The patient-specific fixture according to any one of claims 1 to 11, wherein said positioning element comprises a first hole and a second hole with a different diameter than the first hole, wherein said first hole is configured to allow the insertion of said alignment element, and wherein said second hole is configured to allow the insertion of a fixation element.

13. The patient-specific surgical fixture according to any one of claims 1 to 12, wherein the one or more patient-specific contact elements are further configured to fit onto one or more areas on the glenoid socket by means of anatomy engagement surfaces, each of which at least partially match a surface of bone or cartilage of said one or more areas on the glenoid socket.

14. A method of manufacturing a patient-specific surgical fixture according to any one of claims 1 to 13, comprising the steps of:
a. obtaining volume information of the glenoid socket of the shoulder joint of the patient,
b. obtaining an installation direction of a glenoid socket implant for said patient,
c. identifying and selecting parts of a bone of the shoulder joint which are suitable for inserting an alignment element,
d. identifying and selecting parts of a bone surrounding said implant zone which are suitable for use as a base for a contact surface or surfaces of the patient-specific surgical fixture, and
e. designing and producing the patient-specific surgical fixture based on the information obtained in steps a, b, c and d.

15. The method of manufacturing according to claim 14, wherein at least one of a position or a direction of at least one of said one or more holes is based on information obtained in step c.

## Patentansprüche

1. Patientenspezifische chirurgische Vorrichtung (1) zum Positionieren eines Ausrichtungselements, wie etwa eines Stifts, eines Drahts, einer Schraube oder eines Bohrers, auf oder in der Nähe einer Glenoidpfanne eines Schultergelenks eines Patienten, die Vorrichtung umfassend:
a. eine einteilige Struktur, umfassend ein oder mehrere patientenspezifische Kontaktelemente (2); und
b. ein Positionierungselement (3), das an der einteiligen Struktur starr angebracht ist, wobei es mit einem oder mehreren Löchern (5), die den Einsatz des Ausrichtungselements ermöglichen, versehen ist,
wobei die patientenspezifischen Kontaktelemente konfiguriert sind, um auf mindestens einen zu passen von:
einem oder mehreren Bereichen rund um die Gelenkpfanne oder
einem Rand der Glenoidpfanne,
mittels anatomischer Eingriffsoberflächen, von denen jede mindestens teilweise mit einer Knochen- oder Knorpeloberfläche des mindestens einen übereinstimmt von: dem einen oder den mehreren Bereichen rund um die Glenoidpfanne oder dem Rand der Glenoidpfanne.

2. Patientenspezifische Vorrichtung nach Anspruch 1, wobei mindestens eines des einen oder der mehreren Löcher Teil einer Bohrlehre (9) ist.

3. Patientenspezifische Vorrichtung nach einem der Ansprüche 1 bis 2, wobei das eine oder die mehreren patientenspezifischen Kontaktelemente konfiguriert sind, um zu passen auf den mindestens einen von: dem einen oder den mehreren Bereichen um die Glenoidpfanne oder auf den Rand der Glenoidpfanne an mindestens drei Kontaktpunkten, wobei ein Winkel zwischen einer Linie, die zwischen einem Kontaktpunkt und einem Mittelpunkt eines Kreises oder einer Ellipse, der/die am besten zu dem Rand oder einem im Wesentlichen kreisförmigen Abschnitt des Randes der Glenoidpfanne passt, gezogen wird, und einer Linie, die zwischen einem angrenzenden Kontaktpunkt und dem Mittelpunkt gezogen wird, niemals größer als 180° ist.

4. Patientenspezifische Vorrichtung nach einem der Ansprüche 1 bis 3, wobei eine Oberfläche mindestens eines des einen oder der mehreren patientenspezifischen Kontaktelemente konfiguriert ist, um einer Oberfläche eines Rabenschnabelfortsatzes des Schultergelenks derart zu entsprechen, dass, wenn die anatomische Eingriffsoberfläche des mindestens einen des einen oder der mehreren patientenspezifischen Kontaktelemente auf den Rabenschnabelfortsatz gepasst wird, um mit der Oberfläche des Rabenschnabelfortsatzes in Eingriff zu stehen, die Oberfläche des Rabenschnabelfortsatzes eine Drehung der patientenspezifischen chirurgischen Vorrichtung stoppt.

5. Patientenspezifische Vorrichtung nach einem der Ansprüche 1 bis 4, wobei das eine oder die mehreren Löcher konfiguriert sind, um den Einsatz des Ausrichtungselements in die Glenoidpfanne zu ermöglichen.

6. Patientenspezifische Vorrichtung nach Anspruch 5, wobei das eine oder die mehreren Löcher konfiguriert sind, um den Einsatz des Ausrichtungselements mittig in die Glenoidpfanne zu ermöglichen.

7. Patientenspezifische Vorrichtung nach einem der Ansprüche 1 bis 4, wobei das eine oder die mehreren Löcher konfiguriert sind, um den Einsatz des Ausrichtungselements außerhalb der Glenoidpfanne zu ermöglichen.

8. Patientenspezifische Vorrichtung nach einem der Ansprüche 1 bis 7, wobei das Positionierungselement von dem Rest der patientenspezifischen chirurgischen Vorrichtung abnehmbar ist,
wobei optional eine Verbindung zwischen dem Positionierungselement und dem Rest der patientenspezifischen chirurgischen Vorrichtung ein Element, das aus einer Schwalbenschwanzkupplung, Formschlussmerkmalen, einem Stiftsystem und einem Schnappmechanismus ausgewählt ist, umfasst, oder wobei die Verbindung derart angepasst oder geschwächt ist, das das Positionierungselement von dem Rest der patientenspezifischen chirurgischen Vorrichtung durch Brechen der Verbindung mit chirurgischen Schneidinstrumenten abgenommen werden kann.

9. Patientenspezifische Vorrichtung nach einem der Ansprüche 1 bis 8, wobei das eine oder die mehreren patientenspezifischen Kontaktelemente konfiguriert sind, um an mindestens drei Kontaktpunkten auf Bereiche an dem Rand der Glenoidpfanne zu passen, und aneinander irreversibel fixiert sind.

10. Patientenspezifische Vorrichtung nach einem der Ansprüche 1 bis 9, wobei das eine oder die mehreren patientenspezifischen Kontaktelemente mindestens zwei patientenspezifische Kontaktelemente umfassen, und wobei das Positionierungselement einem der mindestens zwei patientenspezifischen Kontaktelemente entspricht.

11. Patientenspezifische Vorrichtung nach einem der Ansprüche 1 bis 10, ferner umfassend eine Verbindungsstruktur, wobei sich mindestens eines der Positionierungselemente oder eines oder mehrere des einen oder der mehreren patientenspezifischen Kontaktelemente von der Verbindungsstruktur erstrecken.

12. Patientenspezifische Vorrichtung nach einem der Ansprüche 1 bis 11, wobei das Positionierungselement ein erstes Loch und ein zweites Loch mit einem anderen Durchmesser als das erste Loch umfasst, wobei das erste Loch konfiguriert ist, um den Einsatz des Ausrichtungselements zu ermöglichen, und wobei das zweite Loch konfiguriert ist, um den Einsatz eines Fixierungselements zu ermöglichen.

13. Patientenspezifische chirurgische Vorrichtung nach einem der Ansprüche 1 bis 12, wobei das eine oder die mehreren patientenspezifischen Kontaktelemente ferner konfiguriert sind, um mittels anatomischer Eingriffsoberflächen, von denen jede mindestens teilweise mit einer Knochen- oder Knorpeloberfläche des einen oder der mehreren Bereiche auf der Glenoidpfanne übereinstimmt, auf einen oder mehrere Bereiche auf der Glenoidpfanne zu passen.

14. Verfahren zum Herstellen einer patientenspezifischen chirurgischen Vorrichtung nach einem der Ansprüche 1 bis 13, umfassend die Schritte:
a. Erhalten von Volumeninformationen über die Glenoidpfanne des Schultergelenks des Patienten,
b. Erhalten einer Einsetzanweisung für ein Glenoidpfannenimplantat für den Patienten,
c. Identifizieren und Auswählen von Teilen eines Knochens des Schultergelenks, die zum Einsetzen eines Ausrichtungselements geeignet sind,
d. Identifizieren und Auswählen von Teilen eines Knochens, die die Implantatzone umgeben, die zur Verwendung als eine Basis für eine Kontaktfläche oder Oberflächen der patientenspezifischen chirurgischen Vorrichtung geeignet sind, und
e. Entwerfen und Produzieren der patientenspezifischen chirurgischen Vorrichtung basierend auf den Informationen, die in den Schritten a, b, c und d erhalten werden.

15. Herstellungsverfahren nach Anspruch 14, wobei mindestens eine von einer Position und/oder einer Richtung mindestens eines des einen oder der mehreren Löcher auf Informationen, die in Schritt c erhalten werden, basiert.

## Revendications

1. Dispositif chirurgical spécifique au patient (1) servant à positionner un élément d'alignement, tel qu'une broche, un fil, une vis ou une mèche, sur ou à proximité d'une cavité glénoïde de l'articulation de l'épaule d'un patient, ledit dispositif comprenant :
a. une structure monobloc comprenant un ou plusieurs éléments de contact spécifiques au patient (2) ; et
b. un élément de positionnement (3) rigidement attaché à ladite structure monobloc, pourvu d'un ou de plusieurs trous (5) qui permettent l'insertion dudit élément d'alignement,
dans lequel les éléments de contact spécifiques au patient sont conçus pour s'ajuster sur au moins l'un parmi :
une ou plusieurs régions autour de la cavité glénoïde, ou
un rebord de la cavité glénoïde,
au moyen de surfaces de prise anatomiques dont chacune correspond au moins partiellement à une surface d'os ou de cartilage desdits au moins l'un parmi : ladite ou lesdites régions autour de la cavité glénoïde, ou ledit rebord de la cavité glénoïde.

2. Dispositif spécifique au patient selon la revendication 1, dans lequel au moins l'un dudit ou desdits trous fait partie d'un guide de perçage (9).

3. Dispositif spécifique au patient selon l'une quelconque des revendications 1 à 2, dans lequel le ou les éléments de contact spécifiques au patient sont conçus pour s'ajuster sur au moins l'une parmi : ladite ou lesdites régions autour de ladite cavité glénoïde, ou sur le rebord de ladite cavité glénoïde en au moins trois points de contact, dans lequel un angle entre une ligne tracée entre un point de contact et un centre d'un cercle ou d'une ellipse s'ajustant le mieux sur le rebord ou sur une section sensiblement circulaire du rebord de ladite cavité glénoïde et une ligne tracée entre un point de contact adjacent et ledit centre n'est jamais supérieur à 180°.

4. Dispositif spécifique au patient selon l'une quelconque des revendications 1 à 3, dans lequel une surface d'au moins l'un parmi ledit ou lesdits éléments de contact spécifiques au patient est conçu pour correspondre à une surface d'une apophyse coracoïde de l'articulation de l'épaule de telle sorte que, lorsque la surface de prise anatomique de l'au moins un parmi ledit ou lesdits éléments de contact spécifiques au patient est ajustée sur l'apophyse coracoïde de manière à entrer en prise avec la surface de ladite apophyse coracoïde, la surface de l'apophyse coracoïde arrête une rotation du dispositif chirurgical spécifique au patient.

5. Dispositif spécifique au patient selon l'une quelconque des revendications 1 à 4, dans lequel le ou les trous sont conçus pour permettre l'insertion dudit élément d'alignement dans ladite cavité glénoïde.

6. Dispositif spécifique au patient selon la revendication 5, dans lequel le ou les trous sont conçus pour permettre l'insertion dudit élément d'alignement au centre de ladite cavité glénoïde.

7. Dispositif spécifique au patient selon l'une quelconque des revendications 1 à 4, dans lequel le ou les trous sont conçus pour permettre l'insertion dudit élément d'alignement à l'extérieur de ladite cavité glénoïde.

8. Dispositif spécifique au patient selon l'une quelconque des revendications 1 à 7, dans lequel ledit élément de positionnement est détachable du reste dudit dispositif chirurgical spécifique au patient,
dans lequel, éventuellement, une liaison entre ledit élément de positionnement et le reste dudit dispositif chirurgical spécifique au patient comprend un élément choisi parmi un accouplement en queue d'aronde, des caractéristiques de verrouillage, un système de goupilles et un mécanisme d'encliquetage ou dans lequel ladite liaison est adaptée ou affaiblie, de telle sorte que l'élément de positionnement peut être détaché du reste dudit dispositif chirurgical spécifique au patient en rompant ladite liaison avec des instruments chirurgicaux de coupe.

9. Dispositif spécifique au patient selon l'une quelconque des revendications 1 à 8, dans lequel le ou les éléments de contact spécifiques au patient sont conçus pour s'ajuster sur des régions du rebord de ladite cavité glénoïde en au moins trois points de contact et sont irréversiblement fixés l'un à l'autre.

10. Dispositif spécifique au patient selon l'une quelconque des revendications 1 à 9, dans lequel le ou les éléments de contact spécifiques au patient comprennent au moins deux éléments de contact spécifiques au patient et dans lequel ledit élément de positionnement correspond à l'un desdits au moins deux éléments de contact spécifiques au patient.

11. Dispositif spécifique au patient selon l'une quelconque des revendications 1 à 10, comprenant en outre une structure de liaison, dans lequel au moins l'un parmi ledit élément de positionnement ou un ou plusieurs dudit ou desdits éléments de contact spécifiques au patient s'étendent à partir de ladite structure de liaison.

12. Dispositif spécifique au patient selon l'une quelconque des revendications 1 à 11, dans lequel ledit élément de positionnement comprend un premier trou et un second trou avec un diamètre différent du premier trou, dans lequel ledit premier trou est conçu pour permettre l'insertion dudit élément d'alignement, et dans lequel ledit second trou est conçu pour permettre l'insertion d'un élément de fixation.

13. Dispositif chirurgical spécifique au patient selon l'une quelconque des revendications 1 à 12, dans lequel le ou les éléments de contact spécifiques au patient sont en outre conçus pour s'ajuster sur une ou plusieurs régions de la cavité glénoïde au moyen de surfaces de prise anatomiques, dont chacune correspond au moins partiellement à une surface d'os ou de cartilage de ladite ou desdites régions de la cavité glénoïde.

14. Procédé de fabrication d'un dispositif chirurgical spécifique à un patient selon l'une quelconque des revendications 1 à 13, comprenant les étapes consistant à :
a. obtenir des informations de volume de la cavité glénoïde de l'articulation de l'épaule du patient,
b. obtenir une direction d'installation d'un implant de cavité glénoïde pour ledit patient,
c. identifier et sélectionner des parties d'un os de l'articulation de l'épaule qui conviennent à l'insertion d'un élément d'alignement,
d. identifier et sélectionner les parties d'un os entourant ladite zone d'implant qui conviennent pour servir de base à une ou plusieurs surfaces de contact du dispositif chirurgical spécifique au patient, et
e. créer et produire le dispositif chirurgical spécifique au patient sur la base des informations obtenues aux étapes a, b, c et d.

15. Procédé de fabrication selon la revendication 14, dans lequel au moins l'une parmi une position ou une direction d'au moins l'un parmi ledit ou lesdits trous est basée sur les informations obtenues à l'étape c.
